# EUROPÄISCHE PATENTSCHRIFT

(11) **EP 0 602 515 B1**
(45) Veröffentlichungstag und Bekanntmachung des Hinweises auf die Patenterteilung: **15.07.1998**
(21) Anmeldenummer: 93119734.7
(22) Anmeldetag: 08.12.1993
(51) Int. Cl.: C07D 307/32, C07D 207/26, C07D 201/02

(54) **Verfahren zur Herstellung von 5-Ringheterocyclen**
Process for the preparation of 5-membered heterocycles
Procédé de préparation d'hétérocycles pentagonaux

(30) Priorität: 16.12.1992 DE 4242451
(43) Veröffentlichungstag der Anmeldung: 22.06.1994
(73) Patentinhaber: BASF Aktiengesellschaft, 67063 Ludwigshafen (DE)
(72) Erfinder: Fischer, Rolf, Dr., D-69121 Heidelberg (DE); Pinkos, Rolf, Dr., D-67098 Bad Duerkheim (DE)

(56) Entgegenhaltungen:
- EP-A- 0 246 581
- EP-A- 0 525 506
- FR-A- 2 348 910
- CHEMICAL ABSTRACTS, vol. 117, no. 17, 26. Oktober 1992, Columbus, Ohio, US; abstract no. 170939q, KANEDA, MITSUHIRO ET AL. 'Preparation of alkylated aromatic compounds'
- JOURNAL OF THE CHEMICAL SOCIETY, CHEMICAL COMMUNICATIONS Nr. 13 , 1972 Seiten 892 - 893 G.H. POSNER 'A useful method for alpha-methylation of gamma-butyrolactones.'
- JOURNAL OF THE CHEMICAL SOCIETY, CHEMICAL COMMUNICATIONS Nr. 18 , 1973 Seiten 711 - 712 J.L. HERRMANN 'Method for alkylating lactones.'
- JOURNAL OF ORGANIC CHEMISTRY Bd. 31, Nr. 3 , 1966 , EASTON US Seiten 982 - 983 P.G. GASSMAN 'Synthesis of beta-substituted alkylamines via alkylation of N,N-disubstituted amides'

## Beschreibung

Die vorliegende Anmeldung betrifft ein Verfahren zur Herstellung von 5-Ringheterocyclen durch Umsetzung entsprechender in alpha-Position zur Carbonylgruppe Wasserstoff tragenden 5-Ringheterocyclen mit Dimethyl- oder Ethylencarbonat in Gegenwart von stickstoffenthaltenden Basen als Katalysatoren bei erhöhten Drücken.

Aus J. Chem. Soc., Chem. Commun., Seiten 892 bis 893 (1972) und J. Chem. Soc., Chem. Commun., Seiten 711 bis 712 (1973) ist ein Verfahren zur Herstellung alpha-substituierter Lactone durch Umsetzung von gamma-Butyrolacton und delta-Valerolacton (-78°C) in Tetrahydrofuran mit Lithiumdiisopropylamid und anschließende Alkylierung mit Alkylhalogeniden wie Methyliodid, Ethyliodid oder Allylbromid (-40°C) in Ausbeuten von 80 bis über 90 % bekannt.

Aus J. Org. Chem., 31, 982 bis 983 (1966) ist weiterhin bekannt, N-Methylpyrrolidon mit Natriumamid in flüssigem Ammoniak und Alkylhalogeniden zu l-Methyl-3-alkyl-pyrrolidonen umzusetzen.

Den genannten Methoden ist gemeinsam, daß Alkylhalogenide und teure Lithium- oder Natriumbasen eingesetzt und dementsprechend Lithium- oder Natriumhalogenide als Koppelprodukte in stöchiometrischen Mengen (Salzanfall) erhalten werden.

Aus Pol. J. Chem. 60, 957 bis 959 (1986) ist ein Verfahren zur Herstellung von alpha-Methoxycarbonylbutyrolacton durch Umsetzung von Butyrolacton mit Natriumhydrid und Dimethylcarbonat bekannt.

All diese Umsetzungen sind in technischem Maßstab, vor allem wegen der Notwendigkeit, bei tiefen Temperaturen oder mit hoch reaktiven Hydriden zu arbeiten nicht wirtschaftlich durchführbar.

Der vorliegenden Erfindung lag daher die Aufgabe zugrunde, den zuvor genannten Nachteile abzuhelfen.

Demgemäß wurde ein neues und verbessertes Verfahren zur Herstellung von 5-Ringheterocyclen der allgemeinen Formel I 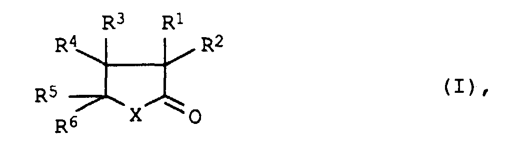 in der
- R¹: Methyl oder Hydroxyethyl,
- R²,R³,R⁴,R⁵,R⁶: Wasserstoff, C₁- bis C₁₂-Alkyl, C₂- bis C₁₂-Alkenyl, Aryl, C₃- bis C₈-Cycloalkyl, C₁- bis C₁₂-Alkoxy, Halogen, C₂- bis C₂₀-Alkoxycarbonyl-alkyl, C₂- bis C₂₀-Alkylcarbonyloxy, Formyl, C₂- bis C₂₀-Formylalkyl, Benzoyl, -CH(OR³) (OR⁵) oder R³ und R⁵ gemeinsam eine gegebenenfalls ein- bis fünffach durch R⁴ substituierte C₂- bis C₇-Alkylenkette oder eine gegebenenfalls ein- bis vierfach durch R⁴ substituierte =CH-CH=CH-CH= Einheit,
- X: Sauerstoff oder N-R⁴
bedeuten, gefunden, welches dadurch gekennzeichnet ist, daß man 5-Ringheterocyclen der allgemeinen Formel II 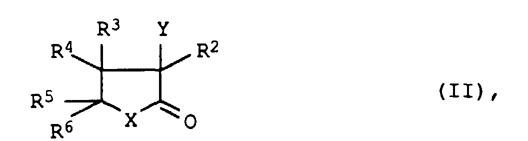 in denen R², R³, R⁴, R⁵, R⁶ und X die obengenannte Bedeutung haben und Y Wasserstoff, Acetyl oder C₂- bis C₂₀-Alkoxycarbonyl bedeutet, mit Dimethyl- oder Ethylencarbonat in Gegenwart einer stickstoffhaltigen Base bei Temperaturen von 50 bis 300°C und Drücken von 0,01 bis 50 bar umsetzt.

Das erfindungsgemäße Verfahren läßt sich wie folgt durchführen:
Man kann die 5-Ringheterocyclen (Lactone und Lactame, speziell Butyrolactone und Pyrrolidone) der allgemeinen Formel II mit Dimethylcarbonat oder Ethylencarbonat in Gegenwart von stickstoffhaltigen Basen z.B. in Druckapparaturen bei Temperaturen von 50 bis 300°C, bevorzugt 100 bis 250°C, besonders bevorzugt 150 bis 230°C und Drücken von 0,01 bis 50 bar, bevorzugt 0,5 bis 5 bar, besonders bevorzugt bei dem sich im jeweiligen Reaktionsgemisch einstellenden Druck umsetzen.

Die Umsetzung kann in der Gasphase, bevorzugt aber in der Flüssigphase diskontinuierlich oder kontinuierlich durchgeführt werden.

Es kann vorteilhaft sein, die Umsetzung in Gegenwart von unter den Reaktionsbedingungen inerten Gasen wie Stickstoff oder Argon durchzuführen.

Setzt man Lactame II, in denen das Wasserstoffatom der NH-Gruppe nicht substituiert ist, mit Dimethylcarbonat oder Ethylencarbonat um, so erfolgt je nach Reaktionsbedingungen neben der Alkylierung in 3-Stellung auch teilweise oder vollständige Alkylierung am Stickstoff.

Die Umsetzung der Lactone und Lactame II in der Flüssigphase kann beispielsweise so durchgeführt werden, daß man ein Gemisch aus II und gegebenenfalls einem Lösungsmittel in Gegenwart von Dimethylcarbonat oder Ethylencarbonat und den stickstoffhaltigen Basen auf die gewünschte Reaktionstemperatur erhitzt. Nach beendeter Reaktion kann das Reaktionsgemisch abgekühlt und zur Gewinnung der gewünschten Lactone bzw Lactame I fraktionierend destilliert werden.

Die erfindungsgemäße Umsetzung kann in Abwesenheit von Lösungsmitteln durchgeführt werden. Es kann jedoch vorteilhaft sein, in Anwesenheit von Lösungsmitteln zu arbeiten. Als Lösungsmittel können beispielsweise acyclische oder cyclische Ether wie Diethylether, Tetrahydrofuran und Dioxan, Aromaten wie Benzol, Toluol und die Xylole, chlorierte Kohlenwasserstoffe wie Chloroform und Methylenchlorid verwendet werden.

Die Menge an Lösungsmittel beträgt, bezogen auf die eingesetzten Lactone und Lactame II, 0 bis 90 Gew.-%, bevorzugt 5 bis 80 Gew.-%, besonders bevorzugt 20 bis 60 Gew.-%.

Das Molverhältnis von Lactonen und Lactamen II zu Dimethylcarbonat bzw. Ethylencarbonat beträgt in der Regel 10 : 1 bis 1 : 1, bevorzugt 5 : 1 bis 2 : 1. Es ist möglich, in überschüssigem Dimethylcarbonat oder Ethylencarbonat als Lösungsmittel zu arbeiten.

Als stickstoffhaltige Basen eignen sich Ammoniak, primäre, sekundäre und tertiäre Amine wie diejenigen der allgemeinen Formel III mit aliphatischen, cycloaliphatischen heteroaromatischen, und/ oder araliphatischen Substituenten. Dabei können zwei aliphatische Substituenten auch zu einem Ring geschlossen sein. Geeignet sind auch Diamine.

Beispiele hierfür sind:
- Ammoniak,
- Methylamin, Ethylamin, Hexylamin und Cyclohexylamin,
- Dimethylamin, Diethylamin, Dibutylamin und Dicyclohexylamin,
- Trimethylamin, Dimethylethylamin, Triethylamin, Tri-n-propylamin, Triisopropylamin, Tributylamin, Trioctylamin, Tricyclohexylamin, Trihexadecylamin, Tricyclohexylamin, Diphenylmethylamin, Dimethylbenzylamin, Dibenzylmethylamin, Tribenzylamin, N.N-Tetramethylhexamethylendiamin, Hexamethylendiamin und Tetramethylendiamin,
- 4-Dimethylaminopyridin, Urotropin, Piperidin, N-Methylpiperidin, Pyrrolidin, N-Methylpyrrolidin, Hexamethylenimin, N-Ethylhexamethylenimin, N-Methylimidazol, 1.4-Diazabicyclo[4.3.0]octan (DABCO), Morpholin, Piperazin und Pyrrolidin.

Weiterhin eignen sich Amidine wie 1.5-Diazabicyclo[4.3.0]non-5-en (DBN), 1.8-Diazabicyclo-[5.4.0]undec-7-en (DBU) und Guanidin.

Bevorzugt sind die tertiären Amine, besonders bevorzugt C₁- bis C₈-Trialkylamine.

Das Molverhältnis von Lactonen und Lactamen II zu den stickstofhaltigen Basen beträgt in der Regel 100 : 1 bis 1 : 1, bevorzugt 20 : 1 bis 3 : 1.

Die als Ausgangsverbindungen verwendeten Butyrolactone und Pyrrolidone der allgemeinen Formel II sind allgemein zugängliche Verbindungen, die z.B. nach Houben-Weyl, Methoden der Organischen Chemie, 4. Auflage, Band VI/2, Seiten 571 bis 759 herstellbar sind.

Geeignete Butyrolactone und Pyrrolidone der allgemeinen Formel II sind z. B. gamma-Butyrolacton, Pyrrolidon, N-Methylpyrrolidon, 4-Methylbutyrolacton, 4.4-Dimethyl-gamma-butyrolacton, 2.3-Dimethylbutyrolacton, 3.4-Dimethylbutyrolacton, 2-(Hydroxyethyl)-butyrolacton, 4-Methyl-2-(hydroxyethyl)-butyrolacton, 4-Chlorbutyrolacton, 3-Methoxybutyrolacton, 3.5-Dimethylpyrrolidon, N-Vinyl-pyrrolidon, N-Cyclohexylpyrrolidon, N-Octylpyrrolidon, 3-Vinylbutyrolacton, 4-Formylbutyrolacton, 4-Methoxycarbonylbutyrolacton, 3-Acetylbutyrolacton, 4-Vinylbutyrolacton, 2-Methoxycarbonylbutyrolacton und 2-Acetylbutyrolacton.

Die Substituenten R¹, R², R³, R⁴, R⁵, R⁶, R⁷, R⁸, R⁹, X und Y, in den Verbindungen I, II und III haben folgende Bedeutungen:
R¹
   - Methyl oder
   - Hydroxyethyl,
R², R³, R⁴, R⁵, R⁶, R⁷, R⁸ und R⁹
   - Wasserstoff,
R², R³, R⁴, R⁵, R⁶, R⁷, R⁸ und R⁹
   - C₃- bis C₈-Cycloalkyl wie Cyclopropyl, Cyclobutyl, Cyclopentyl, Cyclohexyl, Cycloheptyl und Cyclooctyl, bevorzugt C₄-bis C₈-Cycloalkyl wie Cyclobutyl, Cyclopentyl, Cyclohexyl, Cycloheptyl und Cyclooctyl, besonders bevorzugt C₅- bis C₈-Cycloalkyl wie Cyclopentyl, Cyclohexyl und Cyclooctyl,
R²,R³, R⁴, R⁵, R⁶
   - C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-octyl, n-Nonyl, iso-Nonyl, n-Decyl, iso-Decyl, n-Undecyl, iso-Undecyl, n-Dodecyl und iso-Dodecyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.- Butyl,
   - C₂- bis C₁₂-Alkenyl, bevorzugt C₂- bis C₈-Alkenyl wie Vinyl, Allyl, But-2-en-1-yl, But-4-en-1-yl, But-4-en-2-yl, Pent-2-en-1-yl und 2,2-Dimethyl-pent-l-en-1-yl, besonders bevorzugt C₂- bis C₈-Alkenyl wie Vinyl, Allyl, But-2-en-1-yl, But-4-en-1-yl und But-4-en-2-yl,
   - C₁- bis C₁₂-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, n-Nonoxy, iso-Nonoxy, n-Decoxy, iso-Decoxy, n-Undecoxy, iso-Undecoxy, n-Dodecoxy und iso-Dodecoxy, bevorzugt C₁- bis C₈-Alkoxy wie Methoxy, Ethoxy, n-Propoxy, iso-Propoxy, n-Butoxy, iso-Butoxy, sec.-Butoxy, tert.- Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy und iso-Octoxy, besonders bevorzugt C₁- bis C₄-Alkoxy wie Methoxy, Ethoxy, n-Propoxy, iso-Propoxy, n-Butoxy, iso-Butoxy, sec.-Butoxy und tert.-Butoxy, - Halogen wie Fluor, Chlor, Brom und Jod, bevorzugt Fluor, Chlor und Brom, besonders bevorzugt Fluor und Chlor,
   - C₂- bis C₂₀-Alkoxycarbonyl-alkyl, bevorzugt C₂- bis C₈-Alkoxycarbonyl-alkyl wie Methoxycarbonyl-methyl, Methoxycarbonylethyl, Ethoxycarbonyl-methyl und Ethoxycarbonyl-ethyl, besonders bevorzugt Methoxycarbonyl-methyl,
   - C₂- bis C₂₀-Alkylcarbonyloxy, C₂- bis C₈-Alkylcarbonyloxy wie Methylcarbonyloxy, Ethylcarbonyloxy, n-Propylcarbonyloxy und iso-Propylcarbonyloxy, besonders bevorzugt Methoxycarbonyloxy, - Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
   - Formyl,
   - C₂- bis C₂₀-Formyl-alkyl, C₂- bis C₈-Formyl-alkyl wie Formylmethyl und Formyl-ethyl, besonders bevorzugt Formyl-methyl,
   - Benzoyl,
   - -CH(OR³)(OR⁵),
R⁷, R⁸ und R⁹
   - C₁- bis C₃₀-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.- Butyl,
   - C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
R³ und R⁵ oder R⁷ und R⁸
   - gemeinsam
   - eine C₂- bis C₇-Alkylenkette wie -(CH₂)₂-, -(CH₂)₃-, -(CH₂)₄-, -(CH₂)₅-, -(CH₂)₆- und -(CH₂)₇-, bevorzugt -(CH₂)₃- und -(CH₂)₄-, besonders bevorzugt -(CH₂)₄-,
   - eine durch ein bis fünf gleiche oder verschiedene Reste R⁴ substituierte C₂- bis C₇-Alkylenkette, bevorzugt C₃- und C₄-Alkylen, besonders bevorzugt C₄-Alkylen,
R³ und R⁵
   - =CH-CH=CH-CH=,
   - eine durch ein bis fünf gleiche oder verschiedene Reste R⁴ substituierte =CH-CH=CH-CH= Einheit, bevorzugt eine durch einen gleichen oder verschiedenen Rest R⁴ substituierte =CH-CH=CH-CH= Einheit,
X
   - Sauerstoff oder
   - N-R³,
Y
   - Wasserstoff,
   - Acetyl,
   - C₂- bis C₂₀-Alkoxycarbonyl, bevorzugt C₂- bis C₈-Alkoxycarbonyl, besonders bevorzugt C₂- bis C₄-Alkoxycarbonyl wie Methoxycarbonyl, Ethoxycarbonyl, n-Propoxycarbonyl und iso-Propoxycarbonyl.

### Beispiele

### Beispiel 1

Ein Gemisch aus 25 g (250 mmol) 4-Methylbutyrolacton, 112,5 g Dimethylcarbonat und 3 g (50 mmol) Trimethylamin wurde in einem Autoklaven auf 200°C aufgeheizt und 5 Stunden bei dieser Temperatur gerührt. Nach dem Abkühlen erhielt man durch fraktionierte Destillation 18,6 g (74 %) 2,4-Dimethylbutyrolacton; Sdp.: 70 bis 74°C/10 mbar (Selektivität 83%) und 2,3 g nichtumgesetztes gamma-Butyrolacton.

### Beispiel 2

Ein Gemisch aus 21,5 g (250 mmol) gamma-Butyrolacton, 45 g (500 mmol) Dimethylcarbonat und 0,93 g (12,5 mmol) Dimethylethylamin wurde in einem Autoklaven auf 200°C aufgeheizt und 5 Stunden bei dieser Temperatur gerührt. Nach dem Abkühlen erhielt man durch quantitative gaschromatographsiche Analyse des Reaktionsaustrages 13,3 g (53 %) 2-Methylbutyrolacton (Selektivität 74 %) und 6 g nichtumgesetztes gamma-Butyrolacton.

### Beispiele 3 bis 14

Gemische aus 21,5 g gamma-Butyrolacton, 112,5 g Dimethylcarbonat und 5 Mol.-% (bezogen auf eingesetztes gamma-Butyrolacton) verschiedener Stickstoffbasen wurden in Autoklaven 5 Stunden bei 200°C gerührt. Tabelle 1 enthält die Mengen an gaschromatographisch analysiertem 2-Methylbutyrolacton.

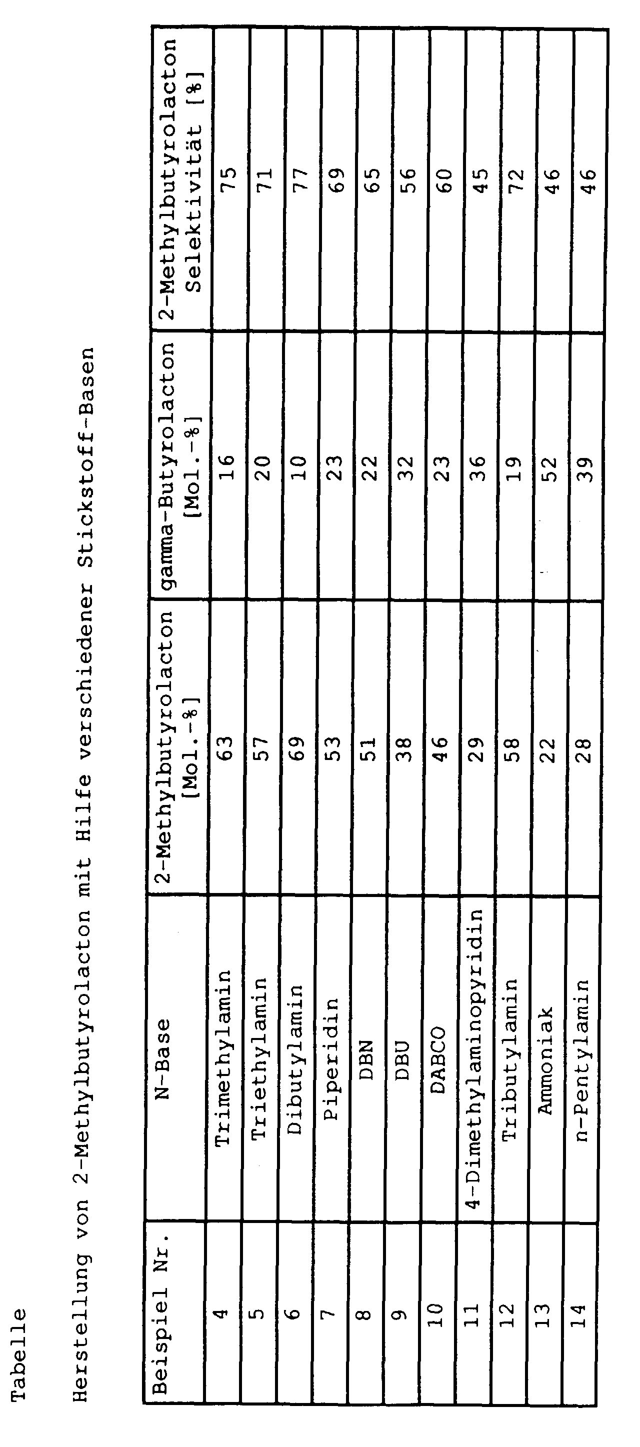

### Beispiel 15

Ein Gemisch aus 25,3 g N-Methylpyrrolidon, 115 g Dimethylcarbonat und 3,7 g Ethyldimethylamin wurde in einem Autoklaven innerhalb einer Stunde auf 200°C aufgeheizt und 5 Stunden bei dieser Temperatur gerührt. Die quantitative gaschromatographische Analyse ergab, daß im Reaktionsaustrag neben 61 Mol-% unumgesetztem N-Methylpyrrolidon 27 Mol-% 2,5-Dimethylpyrrolidon (bezogen auf eingesetztes N-Methylpyrrolidon) enthalten waren. Die 2,5-Dimethylpyrrolidon-Ausbeute beträgt demnach 27 %, die Selektivität 69 %.

### Beispiel 16

Ein Gemisch aus 25,6 g 2-Acetylbutyrolacton, 45 g Dimethylcarbonat und 3 g Ethyldimethylamin wurde innerhalb einer Stunde auf 200°C aufgeheizt und 5 Stunden bei dieser Temperatur gerührt. Nach dem Abkühlen wurden durch fraktionierte Destillation 12,9 g 2-Methylbutyrolacton (65 %, bezogen auf eingesetztes 2-Acetylbutyrolacton) erhalten.

### Beispiel 17

Ein Gemisch aus 26,7 g 2-Methoxycarbonylbutyrolacton, 40,5 g Dimethylcarbonat und 0,7 g Ethyldimethylamin wurde wie in Beispiel 16 beschrieben umgesetzt und aufgearbeitet. Dabei wurden 12,3 g 2-Methylbutyrolacton (66 %, bezogen auf eingesetztes 2-Methoxycarbonylbutyrolacton) erhalten.

## Patentansprüche

1. Verfahren zur Herstellung von 5-Ringheterocyclen der allgemeinen Formel I 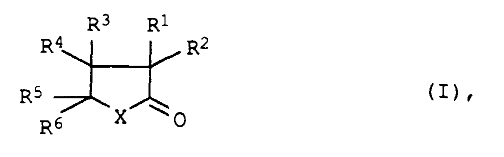 in der
R¹ Methyl oder Hydroxyethyl,
R²,R³,R⁴,R⁵,R⁶ Wasserstoff, C₁- bis C₁₂-Alkyl, C₂- bis C₁₂-Alkenyl, Aryl, C₃- bis C₈-Cycloalkyl, C₁- bis C₁₂-Alkoxy, Halogen, C₂- bis C₂₀-Alkoxycarbonylalkyl, C₂- bis C₂₀-Alkylcarbonyloxy, Formyl, C₂- bis C₂₀-Formylalkyl, Benzoyl, -CH(OR³) (OR⁵) oder R³ und R⁵ gemeinsam eine gegebenenfalls ein- bis fünffach durch R⁴ substituierte C₂- bis C₇-Alkylenkette oder eine gegebenenfalls ein- bis vierfach durch R⁴ substituierte =CH-CH=CH-CH= Einheit,
X Sauerstoff oder N-R⁴
bedeuten, dadurch gekennzeichnet, daß man 5-Ringheterocyclen der allgemeinen Formel II 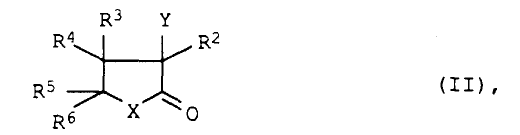 in denen R² R³, R⁴, R⁵, R⁶ und X die obengenannte Bedeutung haben und Y Wasserstoff, Acetyl oder C₂- bis C₂₀-Alkoxycarbonyl bedeutet, mit Dimethyl- oder Ethylencarbonat in Gegenwart einer stickstoffhaltigen Base bei Temperaturen von 50 bis 300°C und Drücken von 0,01 bis 50 bar umsetzt.

2. Verfahren zur Herstellung von 5-Ringheterocyclen der allgemeinen Formel I nach Anspruch 1, dadurch gekennzeichnet, daß R² für Wasserstoff steht.

3. Verfahren zur Herstellung von 5-Ringheterocyclen der allgemeinen Formel I nach Anspruch 1, dadurch gekennzeichnet, daß man als stickstoffhaltigen Base Ammoniak, primäre, sekundäre und tertiäre Amine und/oder Amidine einsetzt.

4. Verfahren zur Herstellung von 5-Ringheterocyclen der allgemeinen Formel I nach Anspruch 1, dadurch gekennzeichnet, daß man die 5-Ringheterocyclen der allgemeinen Formel II zum Dimethylcarbonat oder Ethylencarbonat im Molverhältnis von 10 : 1 bis 1 : 1, bevorzugt 5 : 1 bis 2 : 1 einhält.

5. Verfahren zur Herstellung von 5-Ringheterocyclen der allgemeinen Formel I nach Anspruch 1, dadurch gekennzeichnet, daß man als stickstoffenthaltende Basen der allgemeinen Formel III 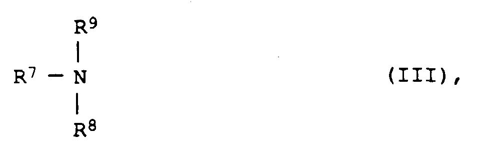 in der
R⁷, R⁸ und R⁹ Wasserstoff, C₁- bis C₃₀-Alkyl, C₃- bis C₈-Cycloalkyl oder C₇- bis C₂₀-Aralkyl bedeuten oder R⁷ und R⁸ eine gegebenenfalls ein- bis fünffach durch R³ C₂- bis C₇-Alkylenkette bilden,
einsetzt.

6. Verfahren zur Herstellung von 5-Ringheterocyclen der allgemeinen Formel I nach Anspruch 1, dadurch gekennzeichnet, daß man als stickstoffhaltigen Base 1,5-Diazabicyclo-[4.3.0]non-5-en (DBN) und 1.8-Diazabicyclo[5.4.0]undec-7-en (DBU) einsetzt.

7. Verfahren zur Herstellung von 5-Ringheterocyclen der allgemeinen Formel I nach Anspruch 1, dadurch gekennzeichnet, daß man das Lacton bzw das Lactam II zur stickstofhaltigen Base im Molverhältnis von 100 : 1 bis 1 : 1, insbesondere 20 : 1 bis 3 : 1 einsetzt.

8. Verfahren zur Herstellung von 5-Ringheterocyclen der allgemeinen Formel I nach Anspruch 1, dadurch gekennzeichnet, daß man die Umsetzung bei Temperaturen von 100 bis 250°C, insbesondere 150 bis 230°C durchführt.

## Claims

1. A process for preparing 5-membered ring heterocycles of the general formula I 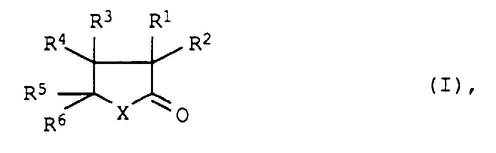 where
R¹ is methyl or hydroxyethyl,
R²,R³,R⁴,R⁵ and R⁶ are hydrogen, C₁- to C₁₂-alkyl, C₂- to C₁₂-alkenyl, aryl, C₃- to C₈-cycloalkyl, C₁- to C₁₂-alkoxy, halogen, C₂- to C₂₀-alkoxycarbonylalkyl, C₂- to C₂₀-alkylcarbonyloxy, formyl, C₂- to C₂₀-formylalkyl, benzoyl or -CH(OR³) (OR⁵), or R³ and R⁵ together are a C₂- to C₇-alkylene chain which is unsubstituted or monosubstituted to pentasubstituted by R⁴ or a =CH-CH=CH-CH= unit which is unsubstituted or monosubstituted to tetrasubstituted by R⁴,
X is oxygen or N-R⁴,
which comprises reacting 5-membered ring heterocycles of the general formula II 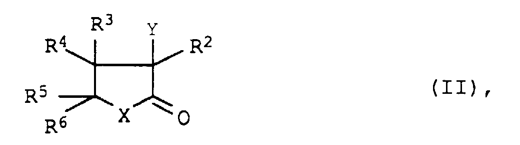 where R² , R³ , R⁴, R⁵, R⁶ and X have the abovementioned meanings and Y is hydrogen, acetyl or C₂- to C₂₀-alkoxycarbonyl, with dimethyl or ethylene carbonate in the presence of a nitrogen-containing base at from 50 to 300°C and from 0.01 to 50 bar.

2. A process for preparing 5-membered ring heterocycles of the general formula I as claimed in claim 1, wherein R² is hydrogen.

3. A process for preparing 5-membered ring heterocycles of the general formula I as claimed in claim 1, wherein the nitrogen-containing base employed is ammonia, primary, secondary and tertiary amines and/or amidines.

4. A process for preparing 5-membered ring heterocycles of the general formula I as claimed in claim 1, wherein the 5-membered ring heterocycles of the general formula II are kept in a molar ratio of 10:1 - 1:1, preferably 5:1 - 2:1, to the dimethyl carbonate or ethylene carbonate.

5. A process for preparing 5-membered ring heterocycles of the general formula I as claimed in claim 1, wherein the nitrogen-containing bases of the general formula III employed are 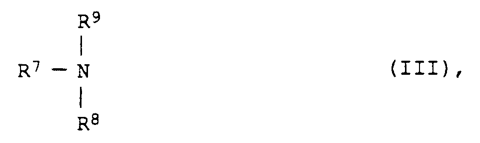 where
R⁷, R⁸ and R⁹ are hydrogen, C₁- to C₃₀-alkyl, C₃- to C₈-cycloalkyl or C₇- to C₂₀-aralkyl, or R⁷ and R⁸ form a C₂- to C₇-alkylene chain which is unsubstituted or monosubstituted to pentasubstituted by R³.

6. A process for preparing 5-membered ring heterocycles of the general formula I as claimed in claim 1, wherein the nitrogen-containing base employed is 1,5-diazabicyclo[4.3.0]non-5-ene (DBN) or 1,8-diazabicyclo[5.4.0]undec-7-ene (DBU).

7. A process for preparing 5-membered ring heterocycles of the general formula I as claimed in claim 1, wherein the lactone or the lactam II is employed in a molar ratio of 100:1 - 1:1, in particular 20:1 - 3:1, to the nitrogen-containing base.

8. A process for preparing 5-membered ring heterocycles of the general formula I as claimed in claim 1, wherein the reaction is carried out at from 100 to 250°C, in particular 150 to 230°C.

## Revendications

1. Procédé de préparation d'hétérocycles à 5 maillons de formule générale I 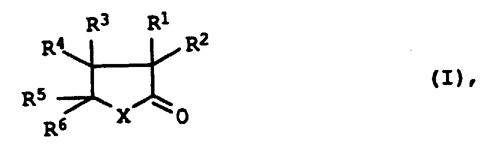 dans laquelle
R¹ représente un groupement méthyle ou hydroxyéthyle,
R², R³, R⁴, R⁵, R⁶ représentent des atomes d'hydrogène, des groupements alkyle en C₁-C₁₂ alcényle en C₂-C₁₂, aryle, cycloalkyle en C₃-C₈, alcoxy en C₁-C₁₂, des atomes d'halogène, des groupements alcoxycarbonylalkyle en C₂-C₂₀, alkylcarbonyloxy en C₂-C₂₀, formyle, formylalkyle en C₂-C₂₀, benzoyle, -CH(OR³) (OR⁵) ou bien R³ et R⁵ représentent ensemble une chaîne alkylène en C₂-C₇ éventuellement substituée une à cinq fois par R⁴, ou bien un motif =CH-CH=CH-CH= éventuellement substitué une à quatre fois par R⁴,
X représente un atome d'oxygène ou N-R⁴
caractérisé en ce que l'on fait réagir des hétérocycles à 5 maillons de formule générale II 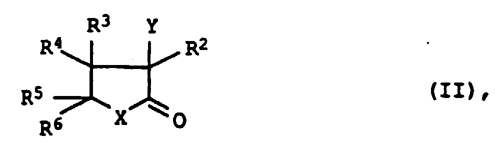 dans laquelle R², R³, R⁴, R⁵, R⁶ et X prennent la signification susmentionnée et Y représente un atome d'hydrogène, un groupement acétyle ou alcoxycarbonyle en C₂-C₂₀, avec du carbonate de diméthyle ou d'éthylène en présence d'une base azotée à des températures de 50-300°C et sous des pressions de 0,01-50 bar.

2. Procédé de préparation d'hétérocycles à 5 maillons de formule générale I selon la revendication 1, caractérisé en ce que R² est mis pour un atome d'hydrogène.

3. Procédé de préparation d'hétérocycles à 5 maillons de formule générale I selon la revendication 1, caractérisé en ce que l'on utilise en tant que base azotée de l'ammoniaque, des amines primaires, secondaires et tertiaires et/ou des amidines.

4. Procédé de préparation d'hétérocycles à 5 maillons de formule générale I selon la revendication 1, caractérisé en ce que l'on ajoute aux hétérocycles à 5 maillons de formule générale II du carbonate de diméthyle ou d'éthylène dans un rapport en moles de 10:1 à 1:1, de préférence 5:1 à 2:1.

5. Procédé de préparation d'hétérocycles à 5 maillons de formule générale I selon la revendication 1, caractérisé en ce que l'on utilise en tant que bases azotées de formule générale III 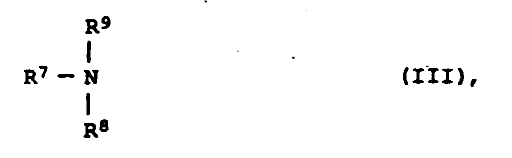 dans laquelle
R⁷, R⁸ et R⁹ représentent des atomes d'hydrogène, des groupements alkyle en C₁-C₃₀, cycloalkyle en C₃-C₈ ou aralkyle en C₇-C₂₀, ou bien R⁷ et R⁸ forment une chaîne alkylène en C₂-C₇, éventuellement substituée une à cinq fois par R³.

6. Procédé de préparation d'hétérocycles à 5 maillons de formule générale I selon la revendication 1, caractérisé en ce que l'on utilise en tant que bases azotées du 1,5-diazabicyclo[4.3.0]non-5-ène (DBN) et du 1,8-diazabicyclo[5.4.0]undéc-7-ène (DBU).

7. Procédé de préparation d'hétérocycles à 5 maillons de formule générale I selon la revendication 1, caractérisé en ce que l'on utilise la lactone, respectivement le lactame II dans un rapport molaire à la base azotée de 100:1 à 1:1, en particulier de 20:1 à 3:1.

8. Procédé de préparation d'hétérocycles à 5 maillons de formule générale I selon la revendication 1, caractérisé en ce que l'on effectue la réaction à des températures de 100-250°C, en particulier de 150-230°C.
